# EUROPEAN PATENT APPLICATION

(11) **EP 4 623 941 A1**
(43) Date of publication of application: **01.10.2025**
(21) Application number: 24208031.5
(22) Date of filing: 22.10.2024
(51) Int. Cl.: A61L 24/00, A61L 24/08

(54) **BIOENERGETIC MIXED TWISTED FILAMENTS AND USES OF THE SAME**

(30) Priority: 28.03.2024 US 202463570958 P
(71) Applicant: China Good International Limited, Kwun Tong (HK)
(72) Inventor: WU, ZHE YU, KWUN TONG RD KWUN TONG KL (TW); WU, JHE-MING, KWUN TONG RD KWUN TONG KL (TW); HO, PO HSIEN, KWUN TONG RD KWUN TONG KL (TW)
(74) Representative: Herrero & Asociados, S.L.

(57) **Abstract**

The present invention relates to a bioenergetic mixed twisted filament and its uses, including bioenergetic yarns, bioenergetic woven fabric, and products manufactured using the bioenergetic mixed twisted filament. The bioenergetic mixed twisted filament comprises a first filament and a second filament twisted with each other. The first filament comprises a first polymer matrix and nanoparticles dispersed within the first polymer matrix. The nanoparticles comprise element(s) selected from the group consisting of Au, Ag, Ti, Ge, Zn, Al, Mg, Si, Cu, Ca, Fe, Ba, K, Na, Mn, Ni, Ga, Pt, and combinations thereof. The second filament has a material different from that of the first filament.

## Description

### CLAIM FOR PRIORITY

This application claims the benefit of U.S. Patent Provisional Application No. 63/570,958 filed on March 28, 2024, the subject matters of which are incorporated herein in their entirety by reference.

### FIELD OF THE INVENTION

The present invention provides bioenergetic mixed twisted filaments and uses thereof, including bioenergetic yarns, bioenergetic woven fabrics, and products made therefrom.

### BACKGROUND OF THE INVENTION

Bleeding is one of the immediate risks associated with open wounds, especially in situations involving heavy bleeding on the battlefield, during emergency first aid, or in surgical procedures. Rapid control of bleeding can effectively prevent the injured person from experiencing hypotension, hypothermia, hemorrhagic shock, or even death. Therefore, to minimize such unfortunate incidents, the industry has been dedicated to developing more effective hemostatic products.

Common hemostatic products include cotton gauze and hemostatic bandages, which can control bleeding from open wounds through manual compression and their inherent liquid absorption capabilities. In recent years, due to the advantages of polysaccharide fibers such as chitosan and alginate, i.e., their superior biocompatibility, biodegradability, and antibacterial properties, these polysaccharide fibers have also been applied in the development of hemostatic products, such as chitosan gauze. However, current hemostatic products continue to face limitations in their hemostatic capabilities.

### SUMMARY OF THE INVENTION

The inventors of the present invention have discovered that hemostatic products made from bioenergetic mixed twisted filaments capable of emitting far infrared radiation (also called bioenergetic radiation here) achieve exceptionally good hemostatic effects. This finding is surprising because far infrared radiation is generally believed to promote blood circulation, which would be detrimental to hemostasis. The inventors also found that by further selecting polysaccharide filaments in combination with far infrared filaments as the twisting materials for the mixed twisted filaments, wound dressing with excellent wound healing effects can be provided, effectively addressing the issue of scarring after wound healing.

Therefore, an object of the present invention is to provide a bioenergetic mixed twisted filament, comprising a first filament and a second filament twisted with each other, wherein:
the first filament comprises a first polymer matrix and nanoparticles dispersed within the first polymer matrix, wherein the nanoparticles comprise element(s) selected from the group consisting of Au, Ag, Ti, Ge, Zn, Al, Mg, Si, Cu, Ca, Fe, Ba, K, Na, Mn, Ni, Ga, Pt, and combinations thereof; and
the second filament has a material different from that of the first filament.

In some embodiments of the present invention, the first filament and the second filament independently have a fiber fineness of 1 denier to 5 denier.

In some embodiments of the present invention, the first filament and the second filament independently have a length of 10 mm to 50 mm.

In some embodiments of the present invention, the first polymer matrix is selected from the group consisting of polyester, poly(ethylene terephthalate) (PET), polyurethane (PU), poly(vinyl chloride) (PVC), poly propylene (PP), polyamide (PA), an amino-containing polymer, silicone, and combinations thereof.

In some embodiments of the present invention, the material of the second filament is selected from the group consisting of plant fiber, animal fiber, regenerated fiber, semi-synthetic fiber, synthetic fiber, and combinations thereof. In some embodiments of the present invention, the material of the second filament is polysaccharide, wherein the polysaccharide is selected from the group consisting of chitosan, cellulose, hydroxymethyl cellulose, collagen, an alginate, and combinations thereof.

In some embodiments of the present invention, a weight ratio of the first filament to the second filament is from 15:85 to 80:20.

Another object of the present invention is to provide a bioenergetic yarn comprising the bioenergetic mixed twisted filament as described above.

Still another object of the present invention is to provide a bioenergetic woven fabric comprising the bioenergetic yarn as described above, wherein the woven fabric may have a knitted structure.

In some embodiments of the present invention, the bioenergetic woven fabric further comprises a metallic yarn.

Still another object of the present invention is to provide a bioenergetic product which is made from the bioenergetic woven fabric as described above.

In some embodiments of the present invention, the bioenergetic product is a hemostatic dressing, wherein the material of the second filament in the bioenergetic mixed twisted filament is cotton or polysaccharide.

In some embodiments of the present invention, the bioenergetic product is a wound dressing, wherein the material of the second filament in the bioenergetic mixed twisted filament is polysaccharide.

Still another object of the present invention is to provide a method for hemostasis, comprising covering a bleeding site of a subject using the hemostatic dressing made from the bioenergetic woven fabric as described above. Examples of the hemostatic dressing include, but are not limited to, hemostatic gauze and a hemostatic bandage.

Still another object of the present invention is to provide a method for promoting wound healing, preventing scar formation and/or fading scar, comprising covering an injury site of a subject using the wound dressing made from the bioenergetic woven fabric as described above. Examples of the wound dressing may be in the form of a band-aid, gauze, an acne patch, or a wound closure strip.

To render the above objectives, technical features, and advantages of the present invention more apparent, the present invention will be described in detail with reference to some embodiments hereinafter.

### BRIEF DESCRIPTION OF THE DRAWINGS

Figs. 1A and 1B are scanning electron microscopy (SEM) images of Au nanoparticles with an SP2 core-shell structure (Fig. 1A: 2,000X magnification; Fig. 1B: 10,000X magnification). The Au nanoparticles were applied to a glass slide via spin coating for observation.
Figs. 2A to 2D are SEM images of Au nanoparticles with an SP2 core-shell structure (Fig. 2A: 9,500X magnification; Fig. 2B: 17,000X magnification; Fig. 2C: 18,000X magnification; Fig. 2D: 55,000X magnification), wherein the Au nanoparticles were applied to a glass slide via drop coating for observation.
Figs. 3A to 3D are SEM images of the first filament containing Au nanoparticles (Fig. 3A: 60X magnification; Fig. 3B: 600X magnification; Fig. 3C: 2,500X magnification; Fig. 3D: 50,000X magnification). The arrows in Figs. 3A and 3C indicate the Au nanoparticles.
Fig. 4 is a picture of the first filament containing Au nanoparticles, which has a fiber fineness of 1.5 denier and a length of 38 mm.
Figs. 5A to 5C are pictures of an embodiment of the bioenergetic yarn of the present invention. The bioenergetic yarn is made from bioenergetic mixed twisted filaments, which are produced by mixing and twisting the first filaments containing Au nanoparticles with chitosan filaments as the second filament.
Fig. 6A is an X-ray image of an embodiment of the bioenergetic woven fabric of the present invention, which contains nine stainless steel yarns.
Figs. 6B to 6D are pictures of an embodiment of the bioenergetic woven fabrics of the present invention, containing three (Fig. 6B), five (Fig. 6C) and eight (Fig. 6D) stainless steel yarns.
Fig. 7 is a picture of an embodiment of the bioenergetic product of the present invention, wherein the bioenergetic product is a hemostatic dressing.

### DESCRIPTION OF THE PREFERRED EMBODIMENT

The following paragraphs will describe some of the embodiments of the present invention in detail. However, the present invention may be embodied in various embodiments and should not be limited to the specific embodiments described in the specification.

Unless otherwise specified herein, the expression "a", "an", "the", or the like recited in the specification and in the claims should include both the singular and plural forms.

The numerical ranges (e.g., 5 to 100) described in this specification encompass all rational numbers within the specified range and any subrange defined by any two rational numbers within that range. Thus, the numerical ranges described in this specification encompass all possible intervals between any two numbers within the listed minimum and maximum values.

Unless otherwise specified, the terms such as "first", "second" or the like recited in this specification and the claims are only used to distinguish different elements or components, but not construed as any special meaning or intended to limit any sequential order.

### 1. Bioenergetic mixed twisted filament

The bioenergetic mixed twisted filament of the present invention comprises a first filament and a second filament, or substantively consists of or consists of the first filament and the second filament.

The fiber fineness of the first filament and the second filament can independently range from 1 denier to 5 denier. For example, the fiber fineness of the first filament and the second filament can be independently 1 denier, 1.5 denier, 2 denier, 2.5 denier, 3 denier, 3.5 denier, 4 denier, 4.5 denier, or 5 denier, or within a range between any two of the values described herein. In a preferred embodiment of the present invention, the first filament and the second filament independently have a fiber fineness ranging from 1.5 denier to 3 denier. For example, the first filament and the second filament may have a fiber fineness of 1.5 denier.

The first filament and the second filament are preferably short fibers. Specifically, the first filament and the second filament can independently have a length ranging from 10 mm to 50 mm. For example, the length of the filament and the second filament can independently be 10 mm, 12 mm, 15 mm, 18 mm, 20 mm, 23 mm, 25 mm, 27 mm, 30 mm, 32 mm, 35 mm, 38 mm, 40 mm, 43 mm, 45 mm, 47 mm or 50 mm, or within a range between any two of the values described herein. In a preferred embodiment of the present invention, the first filament and the second filament independently have a length ranging from 25 mm to 40 mm, such as a length of 38 mm. Without wishing to be bound by any theories, it is believed that the length of the first filament and the second filament within the specified range can achieve the optimal twisting effect.

In the bioenergetic mixed twisted filament of the present invention, the first filament and the second filament can be mixed and twisted in any ratio depending on the need, with no special restrictions. For example, the weight ratio of the first filament to the second filament can range from 1:99 to 99:1, preferably from 15:85 to 80:20. For example, the weight ratio of the first filament to the second filament can be 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 or 95:5, or within a range between any two of the values described herein. For example, the weight ratio of the first filament to the second filament can be 1:4. With the preferred range mentioned above, the bioenergetic mixed twisted filament of the present invention provide enhanced inventive effectiveness. Specifically, hemostatic products made from these bioenergetic mixed twisted filaments achieve superior hemostatic performance, and wound dressings made from a combination of these bioenergetic mixed twisted filaments and polysaccharide filaments can effectively promote wound healing and significantly improve issues related to scarring.

### 1.1 First filament

The bioenergetic mixed twisted filament of the present invention can comprise one or more types of first filaments. The first filament comprises first polymer matrix and nanoparticles dispersed within the first polymer matrix, or substantive consists of or consists of the first polymer matrix and the nanoparticles. The nanoparticles comprise one or more elements selected from the group consisting of Au, Ag, Ti, Ge, Zn, Al, Mg, Si, Cu, Ca, Fe, Ba, K, Na, Mn, Ni, Ga, Pt, and combinations thereof. Preferably, the nanoparticles comprise at least one of Au and Ti.

The nanoparticles used in the present invention can be prepared by the following methods: gas condensation method, liquid phase reduction method and mechanical alloying method. Examples of the liquid phase reduction method include, but are not limited to, co-precipitation method, sol-gel process, micro-emulsion method, hydrothermal/solvothermal synthesis, template method, and biomimetic synthesis. In some embodiments of the present invention, the nanoparticles are prepared by sol-gel process, thereby having a core-shell structure. For example, the nanoparticles with a core-shell structure can be prepared as follows. First, metal precursors containing the desired elements and deionized water are placed in a conical flask, under continuous stirring, the mixture is heated to boiling, and an aqueous solution of sodium citrate is added dropwise to the conical flask during boiling. Then, after continuing to boil for 15 minutes to 25 minutes, the mixture solution in the conical flask is allowed to cool naturally to room temperature. Subsequently, a polyvinylpyrrolidone (PVP) solution is added to the conical flask, and under continuous stirring, the mixture solution is heated again to 60°C to 80°C and maintained for 20 minutes to 40 minutes to obtain a solution containing PVP-coated metal nanocores. Then, sodium citrate and a silicon-containing material are added to the solution containing the PVP-coated metal nanocores, and the pH value of the solution is adjusted to 5 to 7 sodium bicarbonate to react and obtain the desired nanoparticles. In a specific embodiment of the present invention, Au nanoparticles with a core-shell structure are used.

In some embodiments of the present invention, examples of the metal precursors include, but are not limited to, tetrachloroauric acid (HAuCl₄), titanium tetrachloride (TiCl₄), titanium tetraisopropanolate, tetrabutyl titanate, platinic chloride, platinum(II) acetylacetonate, silver nitrate, zinc chloride, zinc nitrate, etc. Examples of the silicon-containing material include, but are not limited to, silanes, siloxanes, silyl ethers, silanols, silanol salts, silyl chloride, and silazole. Examples of silanes include, but are not limited to, methylsilane, methyltrimethoxysilane, methyltriethoxysilane, propyltrimethoxysilane, isobutyltrimethoxysilane, isobutyltriethoxysilane, n-octyltriethoxysilane, vinyltrimethoxysilane, vinyltriethoxysilane, dimethyldimethoxysilane, tetramethoxysilane, tetraethoxysilane (TEOS), ethyltriacetoxysilane, cyclohexylmethyldimethoxysilane, and dicyclopentyldimethoxysilane. Examples of siloxanes include, but are not limited to, polydimethylsiloxane, polymethylhydrogensiloxane (PMHS), polydiethylsiloxane, polymethyl(3-glycidyloxypropyl) siloxane (PMGS), hexamethyldisiloxane, hexamethylcyclotrisiloxane, octamethyltrisiloxane, octamethylcyclotetrasiloxane, and decamethyltetrasiloxane. Examples of silyl ethers include, but are not limited to, trimethylsilyl ether, tert-butyldimethylsilyl ether, and triisopropylsilyl ether. Examples of silanols include, but are not limited to, trimethylsilanol, triethylsilanol, and tri-tert-butylsilanol. In a specific embodiment of the present invention, the silicon-containing material is TEOS.

In some embodiments of the present invention, the first polymer matrix used in the first filament can be selected from the group consisting of polyester, PU, PVC, PP, PA, an amino-containing polymer, silicone, and combinations thereof. In a preferred embodiment of the present invention, the first matrix can be polyester, such as poly(butylene terephthalate) (PBT), poly(ethylene terephthalate) (PET), or a combination thereof.

The first filament can be prepared using conventional methods. Examples of such methods include the full pelletization method, the masterbatch method, and the injection method. In the following examples, the first filament was obtained using the masterbatch method, which involves blending bioenergetic masterbatches containing nanoparticles with polymer matrix masterbatches that do not contain nanoparticles in a certain ratio, followed by extrusion through an extruder and screw spinning.

### 1.2 Second filament

The bioenergetic mixed twisted filament of the present invention can comprise one or more types of second filaments. The material of the second filament is different from that of the first filament. By combining filaments made of different materials, the bioenergetic mixed twisted filament of the present invention can provide excellent composite effects.

The material of the second filament can be a plant fiber, an animal fiber, a regenerated fiber, a semi-synthetic fiber, a synthetic fiber, or a composite fiber containing two or more of the aforementioned types. Depending on the desired composite effect, the material of the second filament can be selected from the group consisting of a polysaccharide, cotton, rayon, silk, heparin, polyester, linen, wool, polyurethane, polyamide (e.g., nylon), polypropylene, and combinations thereof. Examples of polysaccharide include, but are limited to, chitosan, hydroxymethyl cellulose, cellulose, collagen, an alginate, and combinations thereof. In a preferred embodiment of the present invention, the material of the second filament is a polysaccharide. Research has shown that using polysaccharide second filaments in combination with the first filaments can have a synergistic effect, not only effectively promoting wound healing but also minimizing scarring after healing.

### 2. Bioenergetic yarn and woven fabric

The present invention also provides a bioenergetic yarn, which comprises multiple strands of the aforementioned bioenergetic mixed twisted filaments, or substantially consists of or consists of multiple strands of the aforementioned bioenergetic mixed twisted filaments.

The bioenergetic yarn of the present invention exhibits excellent strength and can be used alone or blended with other yarns to form a bioenergetic woven fabric with a woven structure, which offers various advantages. For example, nonwoven fabrics formed by bonding short fibers through thermal pressing often suffer from fiber detachment, which is unacceptable in many applications, especially in hemostatic and wound dressing contexts. In contrast, the fibers in the bioenergetic woven fabric of the present invention are secured by the woven structure, thereby eliminating the issue of fiber detachment. In addition, the woven structure of the bioenergetic woven fabric provides a higher surface area compared to nonwoven fabrics, offering a larger contact area for improved hemostatic effectiveness. The woven structure of the bioenergetic woven fabric also provides better breathability than nonwoven fabrics, which is advantageous for wound dressing applications. In a preferred embodiment of the present invention, the bioenergetic woven fabric has a knitted structure, which is superior in specific surface area, breathability, and stretchability compared to other woven structures.

There are no specific restrictions on the types of other yarns, which may include metallic yarns, non-metallic yarns, or combinations thereof. Examples of metallic yarns include, but are not limited to, composite yarns of metallic fibers and organic fibers, metalized yarns, and pure metallic yarns. Examples of the material of metallic yarns include, but are not limited to, stainless steel, copper, iron, zinc and combinations thereof. Examples of the material of non-metallic yarns include, but are not limited to, cotton, linen, wool, silk, polyester, polyurethane, polyamide (e.g., nylon), polypropylene, polysaccharide, rayon, heparin, and combinations thereof. In an embodiment of the present invention, the bioenergetic woven fabric comprises one or more stainless steel yarns, such as 1, 2, 3, 4, 5, 6, 7, 8, 9, 10, 11 or 12 stainless steel yarns. These stainless steel yarns can be identified by X-rays, making the bioenergetic woven fabric particularly suitable for use as surgical gauze.

### 3. Bioenergetic product obtained by using bioenergetic woven fabrics

The bioenergetic woven fabric of the present invention can be further processed into various bioenergetic products. Examples of the bioenergetic products include, but are not limited to, medical dressings, bedding (e.g., blankets, mattresses, quilt covers, etc.), clothing (e.g., top clothing, pants, underwear, etc.), seat cushions, eye masks, belts, braces (e.g., knee braces, elbow braces, etc.), shawls, and topical patches. Examples of the medical dressings include, but are not limited to, hemostatic dressings and wound dressings. Examples of the hemostatic dressings include, but are not limited to, hemostatic gauze and hemostatic bandages. Examples of the wound dressings include, but are not limited to, band-aids, gauze, acne patches, or wound closure strips.

In an embodiment of the present invention, the bioenergetic woven fabric of the present invention is processed into hemostatic dressing, wherein the second filament in the bioenergetic mixed twisted filament is made of materials having high liquid absorbability, such as cotton or polysaccharide (e.g., chitosan).

In another embodiment of the present invention, the bioenergetic woven fabric of the present invention is processed into wound dressings, wherein the second filament in the bioenergetic mixed twisted filament is preferably made of polysaccharides (e.g., chitosan) to provide a synergistic effect in wound healing.

### 4. Treatment method

As described above, the hemostatic dressing made from the bioenergetic woven fabric of the present invention has surprisingly excellent hemostatic effects, and the wound dressing made from the bioenergetic woven fabric of the present invention can be used on open wounds, providing excellent wound healing effects and minimizing scarring. Therefore, the present invention also provides a method for hemostasis, which comprises covering a bleeding area of an individual with a hemostatic dressing (e.g., hemostatic gauze or hemostatic bandages) made from the bioenergetic woven fabric as described above. In addition, the present invention also provides a method for promoting wound healing, preventing scar formation, and/or reducing scars, which comprises covering an injured area of an individual with a wound dressing (e.g., band-aids, gauze, acne patches, or wound closure strips) made from the bioenergetic woven fabric as described above.

The aforementioned individual can be a human or a non-human vertebrate. Examples of the non-human vertebrate include, but are not limited to, livestock (e.g., cows, horses, sheep, pigs, donkeys and mules), poultries (e.g., chickens, ducks, and gooses), companion animals (e.g., dogs, cats, rabbits, birds, hamsters and guinea pigs), primates (e.g., monkeys, gorillas, and apes), etc.

### 5. Examples

### 5.1 Example 1: Production of bioenergetic woven fabric

### (1-1) Bioenergetic mixed twisted filament

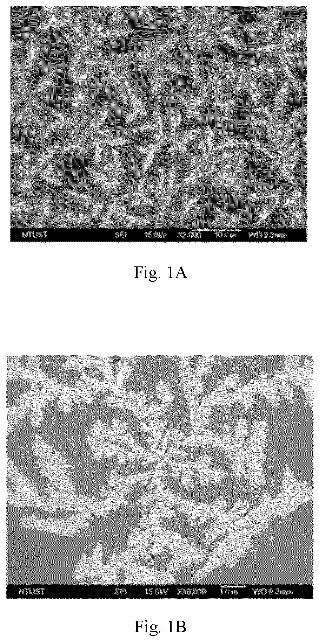

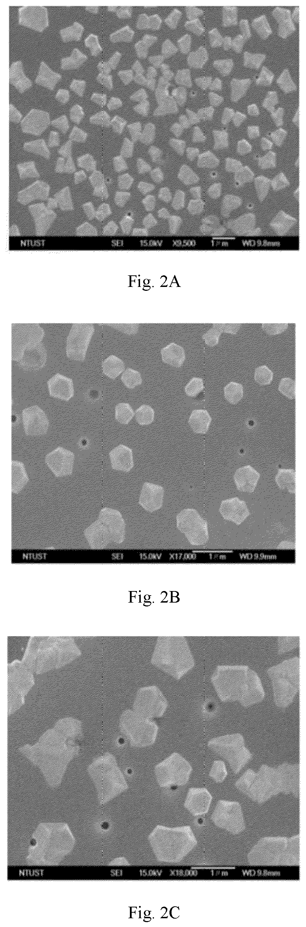

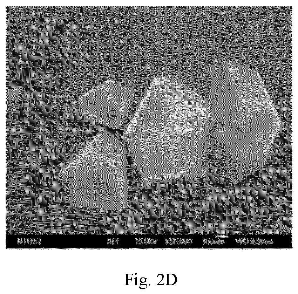

The inventors of the present invention prepared nanoparticles for subsequent experiments through the following two-stage process. In the first stage, tetrachloroauric acid (HAuCl₄) and deionized water were added into a conical flask, which was then heated to boiling under continuous stirring. During boiling, sodium citrate aqueous solution was added dropwise to the conical flask. After boiling for 15 minutes to 25 minutes, the mixture in the conical flask was allowed to cool naturally to room temperature. Then, polyvinylpyrrolidone (PVP) was added into the conical flask, and the mixture was heated again to 60°C to 80°C while continuously stirring for 20 minutes to 40 minutes to obtain a solution containing PVP-coated gold nanocores. In the second stage, a shell structure formed from tetraethoxysilane (TEOS) was prepared to coat the PVP-coated gold nanocores. First, sodium citrate and TEOS were added into a solution containing PVP-coated metal nanocores. The pH value was adjusted to 5 to 7 using sodium bicarbonate, resulting in nanoparticles with an SP2 core-shell structure, where the shell material is TEOS and the core material is gold nanocores, as shown in Figs. 1A, 1B and 2A to 2D.

The nanoparticles, along with and a dispersant and poly(butylene terephthalate) (PBT) were thoroughly mixed using a mixer. The mixture of the nanoparticles, dispersant and PBT was extruded using an extruder at a temperature of 230°C to 295°C to produce far-infrared masterbatches.

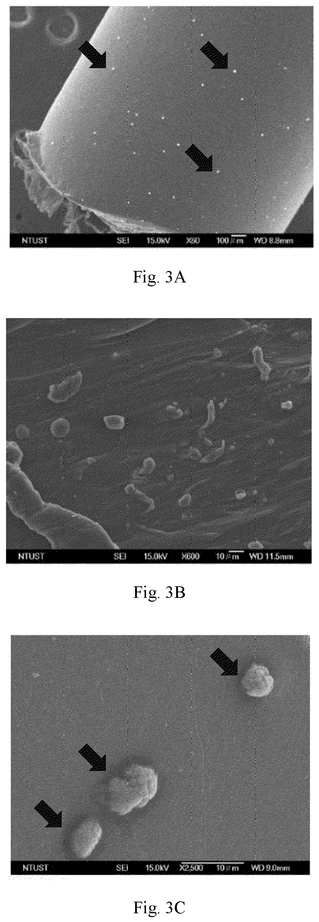

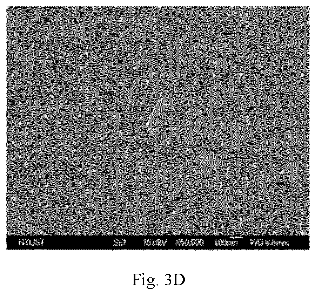

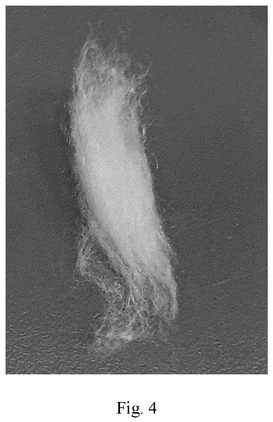

The far-infrared masterbatches were blended with poly(ethylene terephthalate) (PET) masterbatches in a weight ratio of 1:20 (far-infrared masterbatches to PET masterbatches) to obtain a blend. The blend was then extruded using an extruder at a temperature of 265°C. Following extrusion, the blend was subjected to screw spinning to form the first filaments (far-infrared filaments), as shown in Figs. 3A to 3D and 4. The first filaments have a fiber fineness of 1.5 denier and a length of about 38 mm. The arrows in Figs. 3A and 3C indicate the Au nanoparticles.

The first filaments and chitosan filaments (fiber fineness: 1.5 denier; length: 38 mm) as second filaments were mixed and twisted in a weight ratio of 1:4 (the first filaments to chitosan filaments) to obtain bioenergetic mixed twisted filaments.

### (1-2) Bioenergetic yarn

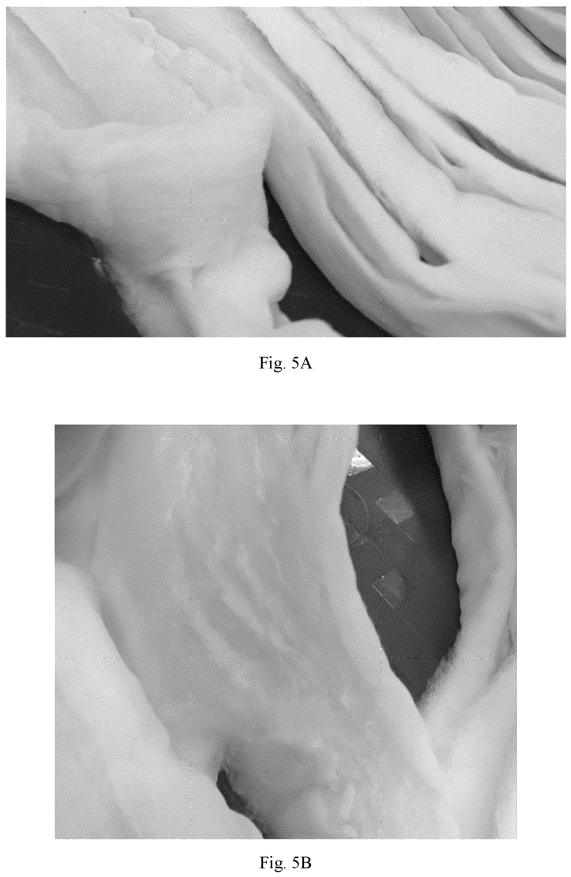

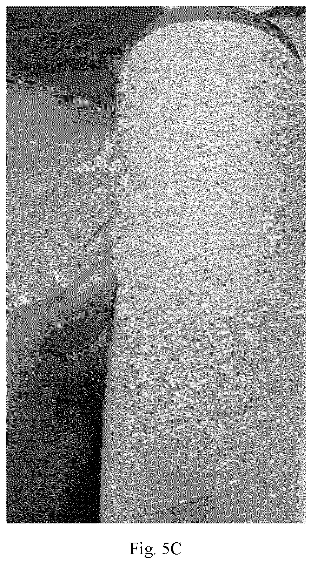

The bioenergetic mixed twisted filaments from (1-1) were spun into bioenergetic yarns, as shown in Figs. 5A to 5C.

### (1-3) Bioenergetic woven fabric

The bioenergetic yarns from (1-2) and stainless steel yarns were knitted using a knitting method to produce a bioenergetic woven fabric containing stainless steel yarns.

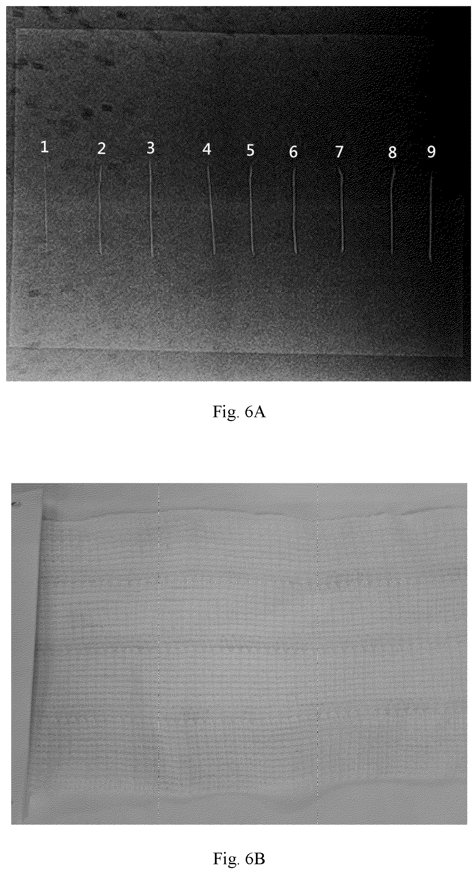

The bioenergetic woven fabric containing stainless steel yarns was examined using an X-ray detector. The results shown in Fig. 6A reveal that the bioenergetic woven fabric containing stainless steel yarns contains nine stainless steel yarns. This demonstrates that the bioenergetic woven fabric containing metallic yarn(s) according the present invention can be identified by an X-ray detector, making it particularly suitable for use as surgical gauze.

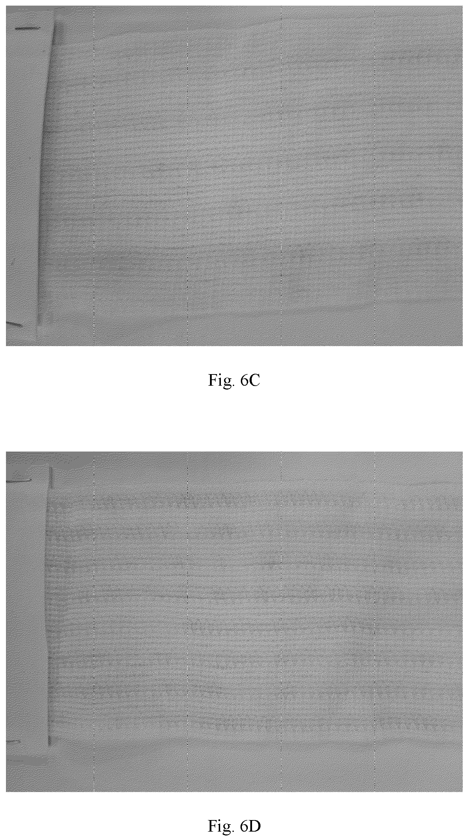

The bioenergetic woven fabrics of the present invention, with varying numbers of stainless steel yarns, are shown in Figs. 6B to 6D (Fig. 6B: three yarns; Fig. 6C: five yarns: Fig. 6D: eight yarns).

### 5.2 Example 2: Hemostasis test

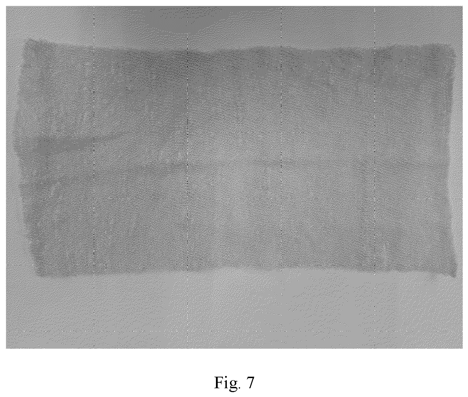

The bioenergetic yarns from (1-2) were woven using a knitting method to produce hemostatic dressings with thickness of 1.2 mm and a thickness of 1 mm, as shown in Fig. 7 (referred to as "GB (thick)" and "GB (thin)", respectively), for subsequent use.

In this example, Sprague-Dawley rats (male, 8-10 weeks of age, 250 g to 300 g body weight per rat) were served as an animal model. These rats were randomly divided into four groups (ten rats per group), and subjected to a hemostasis test using the GB (thick), GB (thin), a commercial chitosan-containing gauze (referred to as "HB"; purchased from Sigma-Aldrich, catalog number: C3646) and regular gauze (purchased from Medline, catalog number: PRM21424C), respectively.

The rats were placed on an operating table and systemically anaesthetized with isoflurane. After confirming that the rats were anaesthetized, a horizontal wound approximately 1 cm in length was made on the right femoral artery using a surgical knife to simulate acute bleeding, and the timer was started. The injured sites were treated with GB (thick), GB (thin), HB or the regular gauze to achieve hemostasis. The hemostatic time for each group of rats was recorded, and the average hemostatic time for each group was calculated. The results are shown in Table 1 below.

**Table 1**

| **Group** | **Average hemostatic time (min)** |
|---|---|
| GB(thick) | 2 |
| GB (thin) | 3 |
| HB | 4 |
| Regular gauze | 24 |

As shown in Table 1, the average hemostatic time of GB (thick) and GB (thin) is only 2 minutes and 3 minutes, respectively, significantly better than that of the commercial chitosan-containing gauze (HB) (about 4 minutes) and the regular gauze (about 24 minutes). These results demonstrate that the hemostatic dressing of the present invention achieves excellent hemostatic effects.

The above embodiments are merely to explain the principles and effects of the present invention and to describe its technical features, and are not intended to limit the scope of the present invention. Any modifications or arrangements that a person skilled in this field can easily achieve without deviating from the principle of the present invention fall within the scope claimed by the present invention. Therefore, the scope of protection of the present invention is defined by the claims appended hereto.

## Claims

1. A bioenergetic mixed twisted filament, comprising a first filament and a second filament twisted with each other, wherein:
the first filament comprises a first polymer matrix and nanoparticles dispersed within the first polymer matrix, wherein the nanoparticles comprise element(s) selected from the group consisting of Au, Ag, Ti, Ge, Zn, Al, Mg, Si, Cu, Ca, Fe, Ba, K, Na, Mn, Ni, Ga, Pt, and combinations thereof; and
the second filament has a material different from that of the first filament.

2. The bioenergetic mixed twisted filament of claim 1, wherein the first filament and the second filament independently have a fiber fineness of 1 denier to 5 denier.

3. The bioenergetic mixed twisted filament of claim 1 or 2, wherein the first filament and the second filament independently have a length of 10 mm to 50 mm.

4. The bioenergetic mixed twisted filament of any one of claims 1-3, wherein the first polymer matrix is selected from the group consisting of polyester, poly(ethylene terephthalate) (PET), polyurethane (PU), poly(vinyl chloride) (PVC), poly propylene (PP), polyamide (PA), an amino-containing polymer, silicone, and combinations thereof.

5. The bioenergetic mixed twisted filament of any one of claims 1-4, wherein the material of the second filament is selected from the group consisting of plant fiber, animal fiber, regenerated fiber, semi-synthetic fiber, synthetic fiber, and combinations thereof.

6. The bioenergetic mixed twisted filament of any one of claims 1-5, wherein the material of the second filament is selected from the group consisting of a polysaccharide, cotton, rayon, silk, heparin, polyester, linen, wool, polyurethane, polyamide, polypropylene, and combinations thereof.

7. The bioenergetic mixed twisted filament of any one of claims 1-6, wherein the material of the second filament is a polysaccharide, and the polysaccharide is preferably selected from the group consisting of chitosan, cellulose, collagen, an alginate, and combinations thereof.

8. The bioenergetic mixed twisted filament of any one of claims 1-7, wherein a weight ratio of the first filament to the second filament is from 15: 85 to 80: 20.

9. A bioenergetic yarn, comprising the bioenergetic mixed twisted filament of any one of claims 1-8.

10. A bioenergetic woven fabric, comprising the bioenergetic yarn of claim 9.

11. The bioenergetic woven fabric of claim 10, which has a knitted structure.

12. The bioenergetic woven fabric of claim 10 or 11, which further comprises a metallic yarn.

13. A bioenergetic product, which is made from the bioenergetic woven fabric of any one of claims 10-12.

14. The bioenergetic product of claim 13, which is a hemostatic dressing, wherein the material of the second filament in the bioenergetic mixed twisted filament is cotton or a polysaccharide.

15. The bioenergetic product of claim 13, which is a wound dressing, wherein the material of the second filament in the bioenergetic mixed twisted filament is a polysaccharide.
